# EUROPEAN PATENT APPLICATION

(11) **EP 0 987 250 A1**
(43) Date of publication of application: **22.03.2000**
(21) Application number: 98904379.9
(22) Date of filing: 19.02.1998
(51) Int. Cl.: C07D 209/22, C07D 209/24, A61K 31/405

(54) **INDOLE DICARBOXYLIC ACID DERIVATIVES**

(30) Priority: 20.02.1997 JP 3598497
(71) Applicant: SHIONOGI & CO., LTD., Osaka 541-0045 (JP)
(72) Inventor: OHTANI, Mitsuaki, Nara-shi, Nara 630-8301 (JP); HAGISHITA, Sanji, Gose-shi, Nara 639-2254 (JP)
(74) Representative: VOSSIUS & PARTNER
(86) International application number: JP9800679
(87) International publication number: WO9837069

(57) **Abstract**

A compound represented by the formula (I): 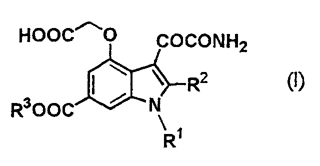 its ester, their pharmaceutically acceptable salt or hydrate thereof and a pharmaceutical composition and a composition for inhibiting sPLA₂ which contain the compound as an active ingredient.

## Description

### Technical Field

The present invention relates to indoledicarboxylic acid derivatives and the pharmaceutical compositions and sPLA₂ inhibitors which contain the said compound as an active ingredient.

### Background Art

sPLA₂ (secretory phospholipase A₂) is an enzyme that hydrolyzes membrane phospholipids and has been considered to be a rate-determining enzyme that governs the so-called arachidonate cascade where arachidonic acid, the hydrolysis product is the starting material. Moreover, lysophospholipids that are produced as by-products in the hydrolysis of phospholipids have been known as important mediators in cardiovascular diseases. Accordingly, in order to normalize excess functions of the arachidonate cascade and the lysophospholipids, it is important to develop compounds which inhibit the sPLA₂-mediated liberation of fatty acids (for example, arachidonic acid), namely, compounds which inhibit the activity or production of sPLA₂. Such compounds are useful for general treatment of symptoms, which are induced and/or sustained by an excess formation of sPLA₂, such as septic shock, adult respiratory distress syndrome, pancreatitis, injury, bronchial asthma, allergic rhinitis, chronic rheumatism, arteriosclerosis, cerebral apoplexy, cerebral infarction, inflammatory colitis, psoriasis, cardiac insufficiency, cardiac infarction, and the like. sPLA₂ participates in many diseases and, besides, its action is potent.

Among a variety of studies of various indole derivatives on application to pharmaceuticals, there is a study indicating that indole derivatives having an oxamoyl group at the 3-position are useful as sPLA₂ inhibitors (JP 7-285933A). However, there is no description about 3-oxamoyl-4-carboxymethyloxyindale derivatives having a carboxyl group or its esterified group at the 6-position of the indole ring.

### Disclosure of Invention

As a result of intensive studies to develop indoledicarboxylic acid derivatives, particularly, 3-oxamoyl-4-carboxymethyloxy-6-carboxylic acid derivatives which possess potent sPLA₂ inhibitory activity and also are highly water-soluble, the present inventors have discovered that the compounds represented by the following formula (I) achieve this objective, thereby accomplishing the present invention.

Thus, the present invention provides a compound represented by the formula (I): 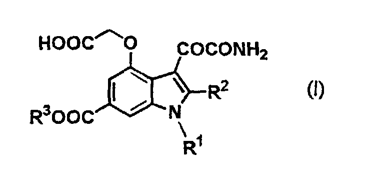 wherein R¹ is optionally substituted alkyl, optionally substituted cycloalkyl, optionally substituted aryl, optionally substituted aralkyl, optionally substituted heteroaryl, or optionally substituted heteroarylalkyl;
R² is hydrogen, optionally substituted alkyl, optionally substituted cycloalkyl, optionally substituted alkenyl, optionally substituted cycloalkenyl, -OR⁴, or -SR⁵ wherein R⁴ and R⁵ are alkyl or cycloalkyl; and
R³ is hydrogen, alkyl, or cycloalkyl, its ester, their pharmaceutically acceptable salt or hydrate thereof. In addition, the present invention provides a pharmaceutical composition and sPLA₂ inhibitor each containing the compound.

Examples of a preferred embodiment of the present invention include compounds in which R³ is hydrogen, methyl, ethyl, n-propyl, isopropyl, or cyclopropyl and, particularly, compounds in which R¹ is aralkyl and R² is alkyl are preferable, where compounds in which R¹ is benzyl and R² is ethyl are most preferable.

Examples of the preferable compounds that represent the present invention include:
(1) [[3-(2-amino-1,2-dioxoethyl)-6-carboxy-2-ethyl-1-benzyl-1H-indol-4-yl]oxy]acetic acid,
(2) [[3-(2-amino-1,2-dioxoethyl)-6-methoxycarbonyl-2-ethyl-1-benzyl-1H-indol-4-yl]oxy]acetic acid,
(3) [[3-(2-amino-1,2-dioxoethyl)-6-ethoxycarbonyl-2-ethyl-1-benzyl-1H-indol-4-yl]oxy]acetic acid,
(4) [[3-(2-amino-1,2-dioxoethyl)-6-n-propoxycarbonyl-2-ethyl-1-benzyl-1H-indol-4-yl]oxy]acetic acid,
(5) [[3-(2-amino-1,2-dioxoethyl)-6-isopropoxycarbonyl-2-ethyl-1-benzyl-1H-indol-4-yl]oxy]acetic acid, and
(6) [[3-(2-amino-1,2-dioxoethyl)-6-cyclopropoxycarbonyl-2-ethyl-1-benzyl-1H-indol-4-yl]oxy]acetic acid.

The term "alkyl" herein used means C₁ to C₁₀ straight or branched chain alkyl. Examples of alkyl are methyl, ethyl, n-propyl, isopropyl, n-butyl, isobutyl, sec-butyl, tert-butyl, n-pentyl, isopentyl, neopentyl, hexyl, isohexyl, n-heptyl, isoheptyl, n-octyl, isooctyl, nonyl, decyl, and the like. Particularly, C₁ to C₄ alkyl is preferred.

The term "cycloalkyl" herein used means C₃ to C₇ cyclic alkyl. Examples of cycloalkyl are cyclopropyl, cyclobutyl, cyclopentyl, cyclohexyl, and cycloheptyl. Particularly, C₃ to C₄ cycloalkyl is preferred.

The term "alkenyl" herein used means C₂ to C₁₀ straight or branched chain alkenyl. Examples of alkenyl are vinyl, propenyl, isopropenyl, butenyl, pentenyl, hexenyl, and the like. Such an alkenyl group has one or more double bond(s) at a possible, arbitrary position.

The term "cycloalkenyl" herein used means C₃ to C₇ cyclic alkenyl. Examples of cycloalkenyl are cyclopropenyl, cyclobutenyl, cyclopentenyl, cyclohexenyl, cycloheptenyl, and the like. Such a cycloalkenyl group has a double bond at a possible, arbitrary position. Particularly, cyclopropen-1-yl, cyclobuten-1-yl, and cyclopenten-3-yl are preferred.

The term "aryl" herein used means phenyl or naphthyl. Examples of aryl are phenyl or naphthyl (e.g., 1-naphthyl or 2-naphthyl). Particularly, phenyl is preferred.

The term "aralkyl" herein used means the above mentioned "alkyl" substituted with the above mentioned "aryl". Examples of aralkyl are benzyl, 2-phenethyl, 1-naphthylmethyl, 2-naphthylmethyl, 1-naphthylethyl, and the like. Particularly, benzyl is preferred.

The term "heteroaryl" herein used means a 5 to 7 membered ring which contains one or more atoms arbitrarily selected from oxygen, sulfur, and/or nitrogen in the ring and may be fused with a carbocyclic ring or another heterocyclic ring. The ring can extend a bond at an arbitrary, substitutable position. Examples of heteroaryl are pyrrolyl (e.g., 1-pyrrolyl), indolyl (e.g., 2-indolyl), carbazolyl (e.g., 3-carbazolyl), imidazolyl (e.g., 1-imidazolyl), pyrazolyl (e.g., 3-pyrazolyl), benzoimidazolyl (e.g., 2-benzoimidazolyl), indazolyl (e.g., 3-indazolyl), pyridyl (e.g., 4-pyridyl), quinolyl (e.g., 8-quinolyl), isoquinolyl (e.g., 3-isoquinolyl), pyrimidinyl (e.g., 4-pyrimidinyl), pyrazinyl (e.g., 2-pyrazinyl), isoxazolyl (e.g., 4-isoxazolyl), oxazolyl (e.g., 2-oxazolyl), furyl (e.g., 2-furyl), benzofuryl (e.g., 3-benzofuryl), thienyl (e.g., 2-thienyl), benzothienyl (e.g., 1-benzothiophen-2-yl and 2-benzothiophen-1-yl), and the like.

The term "heteroarylalkyl" herein used means the above mentioned "alkyl" substituted with the above mentioned "heteroaryl". Examples of heteroaryl are pyridylmethyl (e.g., 4-pyridylmethyl), pyridylethyl (e.g., 1- or 2-(2-pyridyl)ethyl), pyridylpropyl (e.g., 3-(2-pyridyl)propyl), thienylmethyl (e.g., 2-thienylmethyl), quinolylmethyl (e.g., 3-quinolylmethyl), imidazolylmethyl (e.g., 2-imidazolylmethyl), and the like.

Examples of substituents to be used for substitution of each of the above mentioned groups include alkyl (e.g., methyl and ethyl), halogen (e.g., fluorine, chlorine, bromine, and iodine), acyl (e.g., acetyl and benzoyl), alkenyl (e.g., allyl), cycloalkyl (e.g., cyclopropyl), amino, mono- or di-substituted amino (e.g., methylamino and dimethylamino), hydroxy, oxo, alkoxy (e.g., methoxy and ethoxy), cyano, nitro, acyloxy (e.g., acetyloxy), optionally substituted carbamoyl (e.g., carbamoyl, N-methylcarbamoyl), optionally substituted carbamoyloxy (e.g., carbamoyloxy, N-ethylcarbamoyloxy), and the like. These substituents may be substituted at one or more possible positions.

The term "ester" herein used includes methylester, ethylester, n-propylester, isopropylester, or the like.

The compounds of the present invention include the pharmaceutically acceptable salts and hydrates thereof. The pharmaceutically acceptable salts mean salts which are compatible with other ingredients and should not be hazardous to patients. Such examples include salts with alkali metals (lithium, sodium, potassium, and the like), alkaline earth metals (calcium, magnesium, and the like), ammonium, organic bases (triethylamine, pyridine, and the like), amino acids, and the like. These salts can be formed by conventional methods. The hydrates may coordinate with an arbitrary number of water molecules.

### Best Mode for Carrying Out the Invention

The compound of the present invention (I) can be synthesized, for example, by a representative process as embodied in the following, but this embodiment is not intended to restrict the present invention.

### Production Process 1

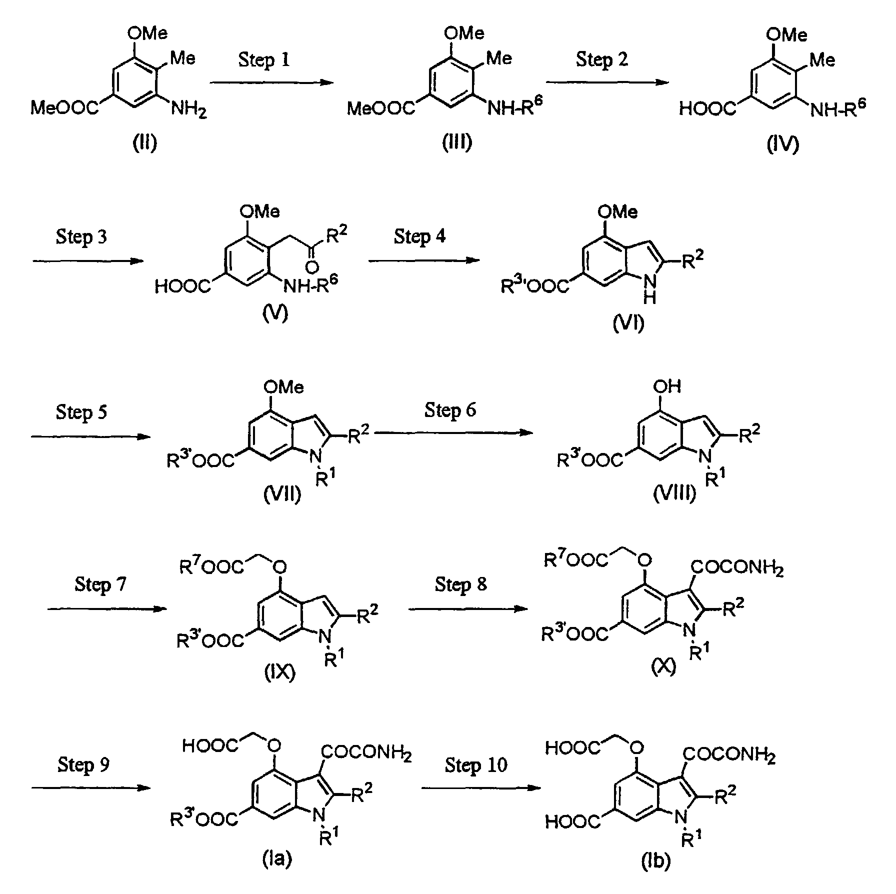

Wherein R¹ and R² are as defined above, R^{3'} is alkyl or cycloalkyl, R⁶ is an amino protecting group, and R⁷ is hydrogen or a carboxyl protecting group.

In the following, each of these steps is described in detail.

### Step 1

In this step, an amino group of the 3-position in compound (II), the starting material, is protected. Compound (II) is a known compound that can be produced by a method described in Constance M. Horris et al., J. Am. Chem. Soc. 1979, 101 (2), 437-445 or by a method well known to those skilled in the art.

Examples of the amino protecting group include carbamate groups (e.g., tert-butyloxycarbonyl and benzyloxycarbonyl), acyl groups (e.g., formyl, acetyl, and benzoyl), sulfonyl groups (e.g., p-toluenesulfonyl), and the like, where tert-butyloxycarbonyl is preferred. Examples of the solvent are tetrahydrofuran, dimethylformamide, chloromethane, dichloromethane, methanol, water, dioxane, and the like.

### Step 2

In this step, the ester group of the 1-position in compound (III) is hydrolyzed. This ester group is hydrolyzed according to conventional methods. The hydrolysis is carried out with an inorganic base in a mixed solvent of water, a lower alcohol, and dimethyl sulfoxide. The lower alcohol is exemplified by methanol, ethanol, or the like. The inorganic base is exemplified by sodium hydroxide, potassium hydroxide, sodium carbonate, potassium carbonate, or the like.

### Step 3

In this step, the 4-methyl group in compound (IV) is acylated. Compound (IV) is reacted with a carboxylic acid derivative in the presence of a strong base. This strong base is exemplified by n-butyllithium, sec-butyllithium, lithium diisopropylamide, sodium amide, potassium amide, potassium tert-butoxide, sodium hydride-dimethyl sulfoxide, or the like. Examples of the reacting carboxylic acid derivative are lower fatty acid chlorides (e.g., propionyl chloride, acetyl chloride, and cyclopropanecarbonyl chloride), acid anhydrides (e.g., propionic anhydride, acetic anhydride, and cyclopropanecarboxylic acid anhydride), acid amides (e.g., N-methoxy-N-methylpropionamide), and the like. The solvent is exemplified by tetrahydrofuran, dimethylformamide, dichloromethane, ether, or the like.

### Step 4

In this step, compound (V) is subjected to ring closure. This ring closure reaction is carried out under an acidic condition using trifluoroacetic acid, sulfuric acid, or the like. In this reaction, the amino protecting group is removed at the same time. The following esterification is carried out according to conventional methods. Examples of this method include acid-catalyzed dehydration reaction with an alcohol, conversion of compound (V) into a mixed anhydride via its acid chloride or acid anhydride followed by condensation with an alcohol, and the like. When this step is carried out in an anhydrous alcohol under an acidic condition with sulfuric acid, the ring closure, the deprotection, and the esterification can be effected simultaneously.

### Step 5

In this step, N-alkylation occurs at the 1-position of the indole ring in compound (VI). The N-alkylation is carried out by reacting compound (VI) with an alkyl halide in the presence of a base. The alkyl halide is a halide with the R¹ group, as exemplified by benzyl bromide, biphenylmethyl bromide, cyclohexyl bromide, cyclohexylmethyl bromide, and the like. The base is exemplified by sodium hydride, potassium hydroxide-tetrabutylammonium bromide, potassium carbonate-potassium iodide, sodium carbonate-potassium iodide, potassium tert-butoxide, or the like.

### Step 6

In this step, the 4-methoxy group in compound (VII) is demthylated. The demethylation reaction is carried out in the presence of Lewis acid. The Lewis acid is exemplified by boron tribromide, ethyl mercaptan-aluminum chloride, iodotrimethylsilane, or the like. Hereupon, it is preferable to repeat the esterification because a small amount of the hydrolyzed compound is formed.

### Step 7

In this step, the 4-hydroxyl group in compound (VIII) is O-alkylated (introduction of an acetic acid residue). The O-alkylation reaction is carried out by reacting compound (VIII) with a monohaloacetic acid derivative (XCH₂COOR⁷: X is halogen and R⁷ is as defined above) in the presence of a base. Examples of the monohaloacetic acid derivative include bromoacetic acid esters, iodoacetic acid esters, and the like. The base is exemplified by sodium hydride, potassium hydroxide-tetrabutylammonium bromide, potassium carbonate-potassium iodide, sodium carbonate-potassium iodide, potassium tert-butoxide, or the like.

### Step 8

In this step, compound (IX) is oxamoylated at the 3-position. Compound (IX) is reacted with a large amount of oxalyl chloride and then the thus-obtained α-ketonic acid chloride is treated with a large amount of ammonia-water.

### Step 9

In this step, the oxyacetic acid ester portion at the 4-position in compound (X) is hydrolyzed. This ester group is hydrolyzed according to conventional methods. The hydrolysis is carried out with an inorganic base in a mixed solvent of water and a lower alcohol. The lower alcohol is exemplified by methanol, ethanol, or the like. The inorganic base is exemplified by sodium hydroxide, potassium hydroxide, sodium carbonate, potassium carbonate, or the like. If desired, moreover, the ester group at the 6-position can be hydrolyzed at the sane time, when strong reaction conditions such as the use of dimethyl sulfoxide or the like as the solvent and/or heating or the like are employed.

### Step 10

In this step, the ester group at the 6-position in compound (Ia) is hydrolyzed in the same manner as in step 2.

### Production Process 2

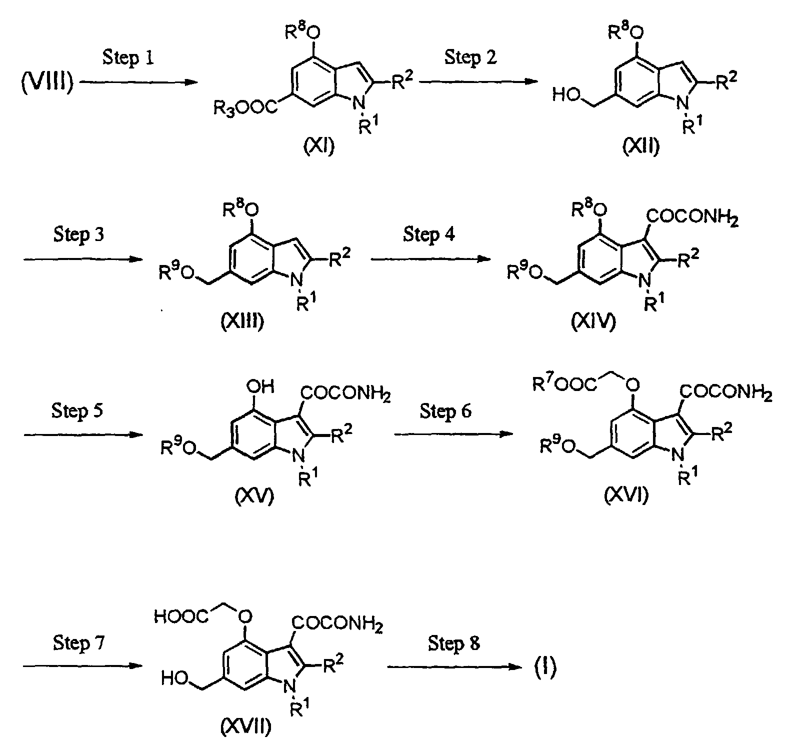

Wherein R¹, R², R³, and R⁷ are as defined above, R⁸ and R⁹ axe a hydroxyl protecting group.

In the following, each of these steps is described in detail.

### Step 1

In this step, the 4-hydroxyl group in compound (VIII) which is obtained in step 6 of Production Process 1 is protected. The hydroxyl protecting group is exemplified by benzyl, allyl, methoxymethyl, tetrahydropyranyl, or the like, where benzyl is preferred. The reaction is carried out in the same manner as employed for the O-alkylation in step 7 of Production Process 1.

### Step 2

In this step, the ester group at the 6-position in compound (XI) is reduced. The hydride reduction is employed for this reduction. In this hydride reduction, a reducing agent such as triethylsilane, lithium aluminum hydride, etc. is used in a solvent such as ether, tetrahydrofuran, or the like.

### Step 3

In this step, the 6-hydroxylmethyl group in compound (XII) is protected. The protecting group is exemplified by an ester group and an ether group. Examples of the reagent include acetic anhydride, acetyl chloride, and the like for the ester protection and dihydrofuran, dihydropyran, methoxymethyl chloride, and the like for the ether protection. Preferably, the ester group is used for the protection and acetyl chloride or acetic anhydride is used for the reagent.

### Step 4

In this step, the 3-position in compound (XIII) is oxamoylated in the same manner as in step 8 of Production Process 1.

### Step 5

In this step, the hydroxyl protecting group at the 4-position in compound (XIV) is removed in the same manner as in step 6 of Production Process 1 or by catalytic reduction or hydrogenolysis according to conventional methods, to achieve the objective easily.

### Step 6

In this step, the 4-hydroxyl group in compound (XV) is O-alkylated in the same manner as in step 7 of Production Process 1.

### Step 7

In this step, the oxyacetate group at the 4-position in compound (XVI) is hydrolyzed in the same manner as in step 9 of Production Process 1.

### Step 8

In this step, the 6-hydroxymetyl group in compound (XVII) is oxidized according to conventional methods.

When a compound contains a functional group(s) possibly interfering the reaction, it can previously be protected and deprotected at an appropriate stage in accordance with the literature such as Protective Groups in Organic Synthesis, Theodora W. Green (John Wiley & Sons).

The compounds represented by the formula (I) are considered to directly inhibit the production of sPLA₂ in mammalian species including the human to exert therapeutic effects, whereby the compounds do not function as antagonists against arachidonic acid and its metabolites and also do not function as inhibitors of 5-lipoxygenase, cyclooxygenase, etc. in the arachidonic acid metabolism in the arachidonate cascade.

Another embodiment of the present invention involves methods which comprise administering compounds illustrated in formula (I) at therapeutically effective doses to mammalian species (including the human) so as to treat sPLA₂-associated diseases such as septic shock, adult dyspnea syndrome, pancreatitis, trauma, bronchial asthma, allergic rhinitis, chronic rheumatism, arteriosclerosis, cerebral apoplexy, cerebral infarction, inflammatory colitis, psoriasis, cardiac insufficiency, cardiac infarction, and the like.

Definite dosages of compounds to be administered in accordance with the present invention so as to achieve sufficient therapeutic effects or preventive effects shall be determined depending on definite factors such as the compound to be administered, the administration route, the conditions of the patient to be treated (sex, age, body weight, and the like), the symptom, and the like. Representative dosages which shall be at harmless dose levels for the active compounds of the present invention are about 0.01 mg/kg/day to about 50 mg/kg/day for injections and about 1 mg/kg/day to about 300 mg/kg/day for oral preparations.

The compound of the present invention can be administered orally or by parenteral routes such as rectal, transdermal, subcutaneous, intravenous, intramuscular, and intranasal administrations. The compound of the present invention may be administered as bulks, but can be better administered as preparations suitable to respective administration routes. Such preparations can be manufactured by compounding (for example, mixing) a compound illustrated in formula (I) in the present invention with pharmaceutically acceptable carrier(s) or diluent(s). The preparations of the present invention can be manufactured by known processes using abundant easily available ingredient(s). Examples of the preparations include tablets, pills, powders, troches, elixirs, suspensions, emulsions, liquids, syrups, aerosols, ointments, and the like.

All of appropriate carriers known to those skilled in the art can be utilized for these preparations. Solid preparations include powders, tablets, capsules, and the like. Solid carrier(s) are one or more substances which are useful as raw materials for perfumaries, lubricants, solubilizers, suspending agents, binding agents, disintegrators, and capsule ingredients. For example, tablets for oral administrations contain disintegrators such as corn starch, alginic acid, and the like and/or binding agents such as gelatin, acacia, and the like as well as lubricants such as magnesium stearate, stearic acid, talc, and the like, together with bulking agents such as calcium carbonate, sodium carbonate, lactose, calcium phosphate, and the like.

Liquid preparations include suspensions, emulsions, syrups, elixirs, and the like. Active ingredients can be dissolved or suspended in pharmaceutically acceptable vehicles such as sterilized water, a sterilized organic solvent, a mixture of them, and the like.

The present invention is embodied in more detail by the following examples, but this embodiment is not intended to restrict the present invention.

The meanings employed in the present examples are indicated below:
Boc: tert-butoxycarbonyl
Bn: benzyl
Ac: acetyl

### Reference Example 1

### N-Methoxy-N-methylpropionamide (19)

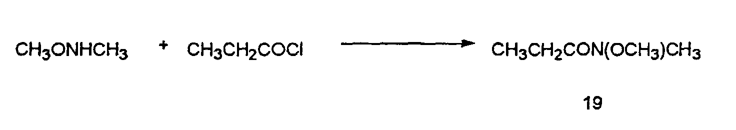

To a suspension of 11.8 g (120 mmol) of N,O-dimethylhydroxylamine hydrochloride in 294 ml of dichloromethane were added at 0°C 33.7 ml (242 mmol) of triethylamine and then a solution of 10.0 ml (115 mmol) of propionyl chloride in dichloromethane (6 ml) and the resulting mixture was stirred at room temperature for 21.5 hours. The reaction mixture was washed successively with water, dilute hydrochloric acid, an aqueous solution of sodium hydrogen carbonate, and an aqueous saturated solution of sodium chloride and was concentrated. The residue was distilled under reduced pressure to obtain 7.42 g (55%) of the objective compound (19).
Boiling point: 67°C (21 mmHg)
¹H NMR (CDCl₃) δ 1.14 (t, J = 7.4 Hz, 3H), 2.45 (q, J = 7.4 Hz, 2H), 3.18 (s, 3H), 3.69 (s, 3H).

### Example 1

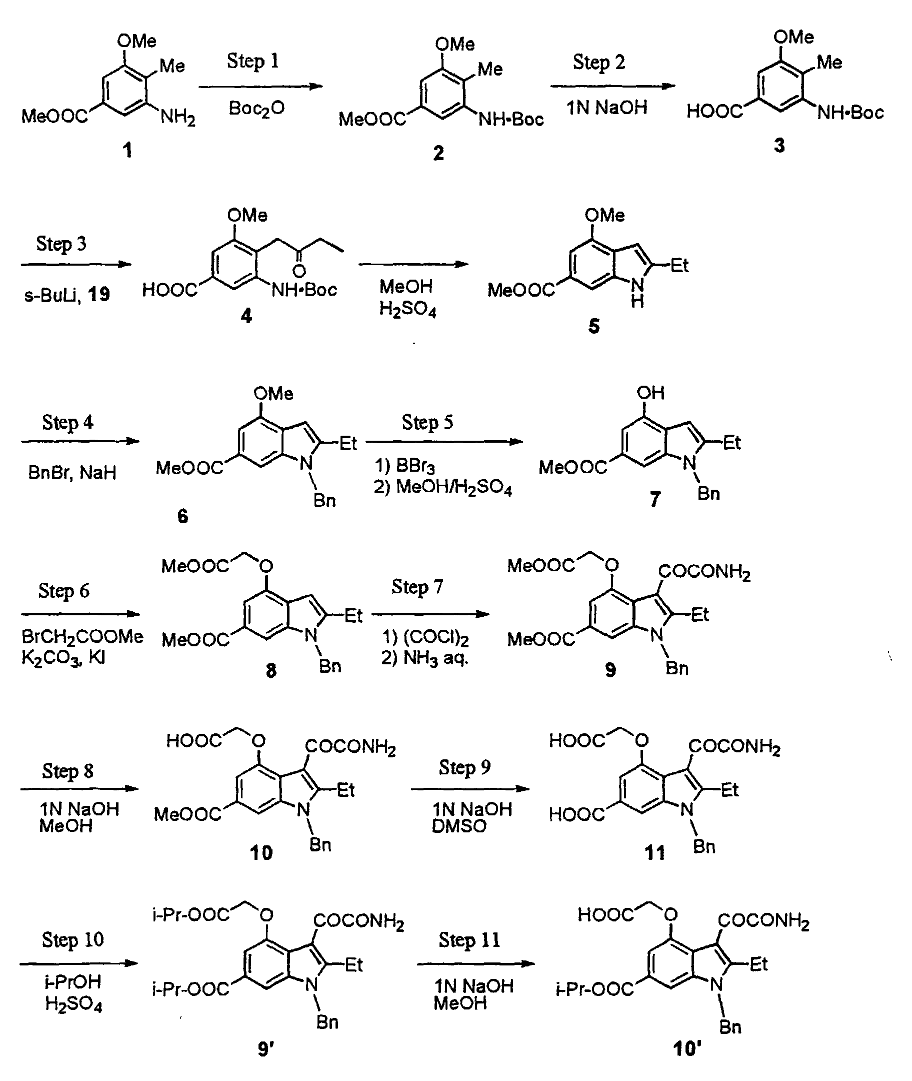

### Step 1

### Synthesis of Methyl 3-tert-Butoxycarbonylamino-5-methoxy-4-methylbenzoate (2)

To a solution of 9.33 g (47.8 mmol) of methyl 3-amino-5-methoxy-4-methylbenzoate (1) known in the literature (J. Am. Chem. Soc. 1979, 101 (2), 437-445) in methanol (150 ml) was added 22.0 ml (95.6 mmol) of di-tert-butyl dicarbonate at room temperature under nitrogen atmosphere and the resulting mixture was stirred for about 74 hours. The reaction solution was concentrated and the thus-obtained crude crystals were recrystallized from ethyl acetate - n-hexane to obtain 13.49 g (96%) of the objective compound (2).
Melting point: 113, 134-136 °C (Dimorphism)
IR ν max (Nujol) 3263, 1717, 1702, 1684, 1591, 1536, 1335 cm⁻¹
¹H NMR (CDCl₃) δ 1.53 (s, 9H), 2.16 (s, 3H), 3.87 (s, 3H), 3.90 (s, 3H), 6.31 (s, 1H), 7.31 (d, J = 1.5 Hz, 1H), 8.11 (s, 1H).

| Elemental analysis (%) for C₁₅H₂₁NO₅: | | | |
|---|---|---|---|
| Calcd.: | C, 61.00; | H, 7.17; | N, 4.74 |
| Found: | C, 60.88; | H, 7.09; | N, 4.73 |

### Step 2

### Synthesis of 3-tert-Butoxycarbonylamino-5-methoxy-4-methylbenzoic acid (3)

To a solution of 13.49 g (45.7 mmol) of compound (2) in a mixed solvent of 180 ml of methanol and 180 ml of dimethyl sulfoxide was added 91.4 ml (91.4 mmol) of 1 N aqueous sodium hydroxide solution under nitrogen atmosphere at room temperature and the resulting mixture was stirred overnight. The reaction solution was poured into dilute hydrochloric acid to make the solution acid and the resulting mixture was extracted with ethyl acetate. The extract was washed successively with water twice and an aqueous saturated solution of sodium chloride, then dried over anhydrous sodium sulfate, and concentrated. The thus-obtained crude crystals were recrystallized from ethyl acetate - n-hexane to obtain 12.48 g (97%) of the objective compound (3).
Melting point (decomp.): 232°C
IR ν max (Nujol) 3343, 2642, 2574, 1701, 1688, 1589, 1530, 1459 cm⁻¹
¹H NMR (CDCl₃) δ 1.46 (s, 9H), 2.06 (s, 3H), 3.83 (s, 3H), 7.25 (s, 1H), 7.59 (s, 1H), 8.71 (s, 1H), 12.36-13.16 (br, 1H).

| Elementary analysis (%) for C₁₄H₁₉NO₅: | | | |
|---|---|---|---|
| Calcd.: | C, 59.78; | H, 6.81; | N, 4.98 |
| Found: | C, 59.52; | H, 6.77; | N, 4.93 |

### Step 3

### Synthesis of 2-Ethyl-4-methoxy-6-methoxycarbonyl-1H-indole (5)

To a solution of 12.48 g (44.4 mmol) of compound (3) in tetrahydrofuran (588 ml) is added dropwise over a period of 25 minutes 146 ml (146 mmol) of a 1.0 M solution of sec-butyllithium in cyclohexane under argon atmosphere at -50°C. After stirring at this temperature for 30 minutes, a solution of 17.15 g (146 mmol) of compound (19) obtained in Reference Example 1 in tetrahydrofuran (18 ml) was added dropwise at - 50°C. After stirring at -50°C to -40°C for 1.5 hours, the reaction mixture was poured into a mixture of ethyl acetate, water, and 40 ml (480 mmol) of concentrated hydrochloric acid to make a partition. The organic layer was washed successively with water twice and an aqueous saturated solution of sodium chloride, then dried over anhydrous sodium sulfate, and concentrated. The thus-obtained crude crystals containing compound (4), 3-tert-butoxycarbonylamino-5-methoxy-4-(2-oxo-n-butyl)benzoic acid, were used, as is, for the next reaction.

To a solution of the above crude crystals containing compound (4) in methanol (500 ml) was gradually added dropwise 6.2 ml (222 mmol) of concentrated sulfuric acid at 0°C. The resulting mixture was stirred at this temperature for 40 minutes and then heated at reflux at 110°C for 14 hours. The reaction mixture was cooled, concentrated, and partitioned between ethyl acetate and water. The organic layer was washed successively with an aqueous solution of sodium hydrogen carbonate, water, and an aqueous saturated solution of sodium chloride, dried over anhydrous sodium sulfate, and then concentrated. The thus-obtained crude crystals were purified by silica gel chromatography (ethyl acetate - n-hexane = 1:4) and then recrystallized from ethyl acetate - n-hexane to obtain 3.02 g (29%) of the objective compound (5).
Melting point: 105-107 °C
IR ν max (Nujol) 3314, 1685, 1589, 1542 cm⁻¹
¹H NMR (CDCl₃) δ 1.36 (t, J = 7.6 Hz, 3H), 2.81 (q, J = 7.6 Hz, 2H), 3.92 (s, 3H), 3.99 (s, 3H), 6.39-6.42 (m, 1H), 7.19 (d, J = 1.0 Hz, 1H), 7.72-7.76 (m, 1H), 8.14 (brs, 1H).

| Elemental analysis (%) for C₁₃H₁₅NO₃: | | | |
|---|---|---|---|
| Calcd.: | C, 66.94; | H, 6.48; | N, 6.00 |
| Found: | C, 66.55; | H, 6.52; | N, 5.94 |

### Step 4

### Synthesis of 1-Benzyl-2-ethyl-4-methoxy-6-methoxycarbonyl-1H-indole (6)

To a solution of 1.80 g (7.72 mmol) of compound (5) in N,N-dimethylformamide (36 ml) was added 339 mg (8.49 mmol) of sodium hydride under nitrogen atmosphere at 0°C. After stirring at this temperature for 10 minutes, 1.0 ml (8.49 mmol) of benzyl bromide was added thereto at 0°C. After stirring at room temperature for 4 hours, the reaction solution was partitioned in a mixture of ethyl acetate and dilute hydrochloric acid. The organic layer was washed successively with water thrice and an aqueous saturated solution of sodium chloride, dried over anhydrous sodium sulfate, and then concentrated. The thus-obtained crude product was subjected to silica gel chromatography (ethyl acetate - n-hexane = 1:9) followed by recrystallization from ethyl acetate - n-hexane to obtain 2.27 g (91%) of the objective compound (6).
Melting point: 114-116 °C
IR ν max (Nujol) 1708, 1583, 1532, 1440, 1219, 1233 cm⁻¹
¹H NMR (CDCl₃) δ 1.31 (t, J= 7.5 Hz, 3H), 2.67 (dq, J = 0.9, 7.5 Hz, 2H), 3.88 (s, 3H), 4.02 (s, 3H), 5.36 (s, 2H), 6.50 (d, J = 0.9 Hz, 1H), 6.89-6.94 (m, 2H), 7.19-7.30 (m, 4H), 7.69 (s, 1H).

| Elemental analysis (%) for C₂₀H₂₁NO₃: | | | |
|---|---|---|---|
| Calcd.: | C, 74.28; | H, 6.55; | N, 4.33 |
| Found: | C, 74.23; | H, 6.60; | N, 4.40 |

### Step 5

### Synthesis of 1-Benzyl-2-ethyl-4-hydroxy-6-methoxycarbonyl-1H-indole (7)

To a solution of 2.05 g (6.34 mmol) of compound (6) in dichloromethane (50 ml) is added dropwise 19.0 ml (19.0 mmol) of a 1 M solution of boron tribromide in dichloromethane under nitrogen atmosphere at -78°C. After stirring at this temperature for one hour and 40 minutes and then at -20°C to -30°C for 6 hours, the reaction solution was cooled to -40°C and mixed with water to make partition. After concentration of the organic layer, 60 mL of methanol and 1.0 mL (36 mmol) of concentrated sulfuric acid were added and the resulting mixture was heated at reflux at 90°C for 14 hours. The reaction mixture was cooled, then concentrated, and partitioned in a mixture of ethyl acetate and water. The organic layer was washed successively with water and an aqueous saturated solution of sodium chloride, then dried over anhydrous sodium sulfate, and concentrated. The thus-obtained crude product was subjected to silica gel chromatography (ethyl acetate - n-hexane = 1:9) followed by recrystallization from ethyl acetate - n-hexane to obtain 1.03 g (53%) of the objective compound (7).
Melting point: 160-162 °C
IR ν max (Nujol) 3433, 1682, 1588, 1533 cm⁻¹
¹H NMR (CDCl₃) δ 1.31 (t, J = 7.5 Hz, 3H), 2.66 (q, J = 7.5 Hz, 2H), 3.87 (s, 3H), 5.34 (s, 2H), 5.89 (s, 1H), 6.48 (s, 1H), 6.88-6.96 (m, 2H), 7.18-7.30 (m, 4H), 7.64 (s, 1H).

| Elemental analysis (%) for C₁₉H₁₉NO₃: | | | |
|---|---|---|---|
| Calcd.: | C, 73.77; | H, 6.19; | N, 4.53 |
| Found: | C, 73.50; | H, 6.25; | N, 4.58 |

### Step 6

### Synthesis of 1-Benzyl-2-ethyl-6-methoxycarbonyl-4-methoxycarbonylmethyloxy-1H-indole (8)

To a solution of 230 mg (743 µmol) of compound (7) in N,N-dimethylformamide (5 ml) were successively added 84 µl (892 µmol) of methyl bromoacetate, 308 mg (2.23 mmol) of anhydrous potassium carbonate, and 25 mg (149 µmol) of powdered potassium iodide under nitrogen atmosphere at room temperature. After stirring at room temperature for 4 hours, the reaction solution was partitioned in a mixture of ethyl acetate and 10% hydrochloric acid. The organic layer was washed successively with water, an aqueous solution of sodium hydrogen carbonate, water, and an aqueous saturated solution of sodium chloride, then dried over anhydrous sodium sulfate, and concentrated. The thus-obtained crude product was recrystallized from ethyl acetate - n-hexane to obtain 253 mg (89%) of the objective compound (8).
Melting point: 108-109 °C
IR ν max (Nujol) 1766, 1711, 1215 cm⁻¹
¹H NMR (CDCl₃) δ 1.32 (t, J = 7.5 Hz, 3H), 2.67 (q, J = 7.5 Hz, 2H), 3.84 (s, 3H), 3.87 (s, 3H), 4.86 (s, 2H), 5.36 (s, 2H), 6.59 (s, 1H), 6.88-6.96 (m, 2H), 7.10-7.13 (m, 1H), 7.19-7.33 (m, 3H), 7.72 (s, 1H).

| Elemental analysis (%) for C₂₂H₂₃NO₅·0.1 H₂O: | | | |
|---|---|---|---|
| Calcd.: | C, 68.95; | H, 6.10; | N, 3.65 |
| Found: | C, 68.88; | H, 6.06; | N, 3.65 |

### Step 7

### Synthesis of 1-Benzyl-2-ethyl-6-methoxycarbonyl-4-methoxycarbonylmethyloxy-3-oxamoyl-1H-indole (9)

To a solution of 220 mg (577 µmol) of compound (8) in tetrahydrofuran (6 ml) was added 101 µl (1.15 mmol) of oxalyl chloride under nitrogen atmosphere at room temperature. After stirring at 50°C for 4 hours, the resulting mixture was cooled to 0°C and then slowly added dropwise into a solution of 386 µl (5.77 mmol) of 28% by weight of aqueous ammonia in tetrahydrofuran (1 ml) at 0°C. After stirring at 0°C for 30 minutes, the reaction mixture was concentrated and the obtained solid was partitioned in a mixture of chloroform and water. The organic layer was washed with water, dried over anhydrous sodium sulfate, and concentrated. The thus-obtained crude product was recrystallized from ethyl acetate - n-hexane to obtain 236 mg (90%) of the objective compound (9).
Melting point: 201-203 °C
IR ν max (Nujol) 3372, 3165, 1731, 1709, 1670, 1642 cm⁻¹
¹H NMR (CDCl₃) δ 1.19 (t J = 7.5 Hz, 3H), 2.93 (q, J = 7.5 Hz, 2H), 3.80 (s, 3H), 3.87 (s, 3H), 4.80 (s, 2H), 5.42 (s, 2H), 5.59 (brs, 1H), 6.63 (brs, 1H), 6.98-7.07 (m, 2H), 7.24-7.36 (m, 4H), 7.70 (d, J = 0.9 Hz, 1H).

| Elemental analysis (%) for C₂₄H₂₄N₂O₇·0.2 H₂O: | | | |
|---|---|---|---|
| Calcd.: | C, 63.21; | H, 5.39; | N, 6.14 |
| Found: | C, 63.13; | H, 5.34; | N, 6.13 |

### Step 8

### Synthesis of 1-Benzyl-4-carboxymethyloxy-2-ethyl-6-methoxycarbonyl-3-oxamoyl-1H-indole (10)

To a suspension of 200 mg (442 µmol) of compound (9) in 4 ml of methanol was added 884 µl (884 µmol) of 1 N aqueous sodium hydroxide solution under nitrogen atmosphere at 0°C. After stirring at room temperature for 5 hours and 40 minutes, the reaction solution was poured into 10% hydrochloric acid. The precipitated crystals were collected by filtration and washed with water to obtain 175 mg (90%) of the objective compound (10).
Melting point (decomp.): 247-251 °C
IR ν max (Nujol) 3404, 3332, 3285, 3226, 1719, 1604 cm⁻¹
¹H NMR (d6-DMSO) δ 1.08 (t, J = 7.5 Hz, 3H), 2.92 (q, J = 7.5 Hz, 2H), 3.81 (s, 3H), 4.73 (s, 2H), 5.65 (s, 2H), 7.01 (d, J = 6.8 Hz, 2H), 7.09 (d, J = 0.9 Hz, 1H), 7.22-7.38 (m, 3H), 7.48 (s, 1H), 7.75 (d, J = 0.6 Hz, 1H), 7.84 (s, 1H).

| Elemental analysis (%) for C₂₃H₂₂N₂O₇•0.3 H₂O: | | | |
|---|---|---|---|
| Calcd.: | C, 62.24; | H, 5.13; | N, 6.31 |
| Found: | C, 62.11; | H, 5.12; | N, 6.14 |

### Step 9

### Synthesis of 1-Benzyl-6-carboxy-4-carboxymethyloxy-2-ethyl-3-oxamoyl-1H-indole (11)

To a solution of 140 mg (319 µmol) of compound (10) in dimethyl sulfoxide (5 ml) was added 960 µl (957 µmol) of 1 N aqueous sodium hydroxide solution under nitrogen atmosphere at room temperature. After stirring at room temperature for 2.5 hours, 1 N hydrochloric acid was added to the reaction solution. The precipitated crystals were collected by filtration and washed with water to obtain 120 mg (88%) of the objective compound (11).
Melting point (decomp.): 267-269 °C
IR ν max (Nujol) 3650, 3420, 3339, 3185, 1724, 1710, 1682, 1658, 1624, 1577 cm⁻¹
¹H NMR (d6-DMSO) δ 1.09 (t, J = 7.1 Hz, 3H), 2.93 (q, J = 7.2 Hz, 2H), 4.71 (s, 2H), 5.63 (s, 2H), 7.02 (d, J = 7.2 Hz, 2H), 7.08 (s, 1H), 7.22-7.38 (m, 3H), 7.48 (s, 1H), 7.71 (s, 1H), 7.83 (s, 1H), 12.85 (brs, 2H).

| Elemental Analysis (%) for C₂₂H₂₀N₂O₇·H₂O: | | | |
|---|---|---|---|
| Calcd.: | C, 59.73; | H, 5.01; | N, 6.33 |
| Found: | C, 59.79; | H, 4.85; | N, 6.33 |

### Step 10

### Synthesis of 1-Benzyl-2-ethyl-6-isopropoxycarbonyl-4-isopropoxycarbonylmethyloxy-3-oxamoyl-1H-indole (9')

To a suspension of 226 mg (533 µmol) of compound (11) in 40 ml of isopropanol was gradually added dropwise 0.1 ml (3.6 mmol) of concentrated sulfuric acid under nitrogen atmosphere at room temperature. The resulting mixture was heated at reflux at 110°C for about 28 hours, cooled, and then partitioned in a mixture of ethyl acetate and water. The organic layer was washed successively with water twice and an aqueous saturated solution of sodium chloride, dried over anhydrous sodium sulfate, and then concentrated. The thus-obtained crude product was subjected to silica gel chromatography (ethyl acetate - n-hexane = 1:4) followed by recrystallization from ethyl acetate - n-hexane to obtain 70 mg (26%) of the objective compound (9').
Melting point: 184-185 °C
¹H NMR (CDCl₃) δ 1.19 (t, J = 7.5 Hz, 3H), 1.33 (d, J = 6.3 Hz, 6H), 2.93 (q, J = 7.5 Hz, 2H), 4.74 (s, 2H), 5.41 (s, 2H), 5.48 (brs, 1H), 6.60 (brs, 1H), 6.99-7.07 (m, 2H), 7.22 (s, 1H), 7.23-7.35 (m, 3H), 7.69 (s, 1H).

### Step 11

### Synthesis of 1-Benzyl-4-carboxymethyloxy-2-ethyl-6-isopropoxycarbonyl-3-oxamoyl-1H-indole (10')

Compound (9') was reacted in the same manner as in step 8 to obtain the objective compound (10'). (Yield: 97%).
Melting point (decomp.): 204 °C
IR ν max (Nujol) 3445, 3332, 1733, 1706, 1627 cm⁻¹
¹H NMR (d6-DMSO) δ 1.08 (t, J = 7.2 Hz, 3H), 1.28 (d, J = 6.3 Hz, 6H), 2.92 (q, J = 7.2 Hz, 2H), 4.69 (s, 2H), 5.01-5.14 (m, 1H), 5.63 (s, 2H), 6.99-7.12 (m, 3H), 7.23-7.39 (m, 3H), 7.48 (s, 1H), 7.71 (s, 1H), 7.86 (s, 1H).

| Elemental analysis (%) for C₂₅H₂₆N₂O₇·0.6 H₂O: | | | |
|---|---|---|---|
| Calcd.: | C, 62.91; | H, 5.74; | N, 5.87 |
| Found: | C, 62.96; | H, 5.51; | N, 5.99 |

### Reference Example 2

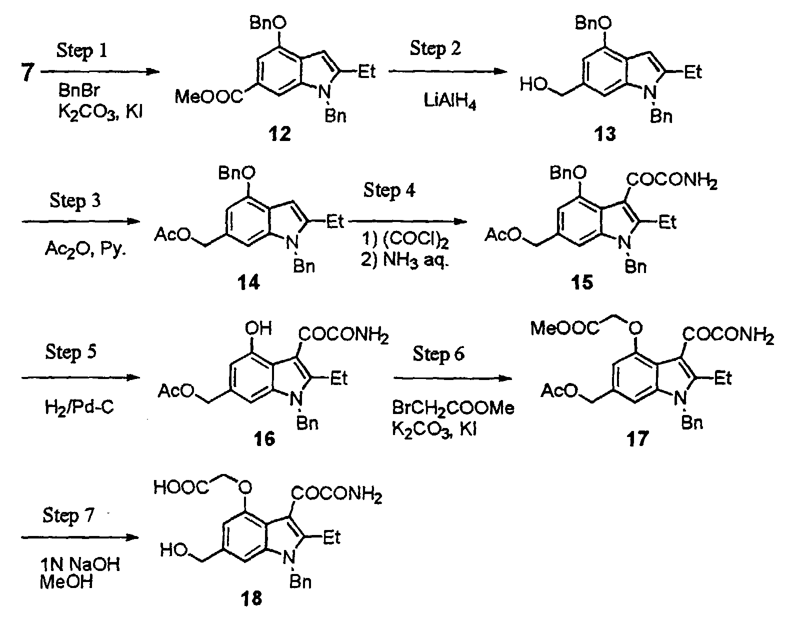

### Step 1

### Synthesis of 1-Benzyl-4-benzyloxy-2-ethyl-6-methoxycarbonyl-1H-indole (12)

To a solution of 1.03 g (3.33 mmol) of compound (7) in N,N-dimethylformamide (20 ml) were successively added 475 µl (4.00 mmol) of benzyl bromide, 1.38 g (9.99 mmol) of anhydrous potassium carbonate, and 111 mg (666 µmol) of powdered potassium iodide under nitrogen atmosphere. After stirring at room temperature overnight the reaction solution was partitioned in a mixture of ethyl acetate and dilute hydrochloric acid. The organic layer was washed successively with water thrice and an aqueous saturated solution of sodium chloride, then dried over anhydrous sodium sulfate, and concentrated. The thus-obtained crude product was recrystallized from ethyl acetate - n-hexane to obtain 1.16 g (87%) of the objective compound (12).
Melting point 157-159 °C
IR ν max (Nujol) 1702, 1577, 1531 cm⁻¹
¹H NMR (CDCl₃) δ 1.31 (t, J = 7.5 Hz, 3H), 2.67 (dq, J = 0.9, 7.5 Hz, 2H), 3.88 (s, 3H), 5.28 (s, 2H), 5.36 (s, 2H), 6.56 (d, J = 0.9 Hz, 1H), 6.90-6.95 (m, 2H), 7.22-7.30 (m, 3H), 7.33-7.48 (m, 4H), 7.51-7.59 (m, 2H), 7.70 (s, 1H).

| Elemental analysis (%) for C₂₆H₂₅NO₃: | | | |
|---|---|---|---|
| Calcd.: | C, 78.17; | H, 6.31; | N, 3.51 |
| Found: | C, 77.93; | H, 6.40; | N, 3.54 |

### Step 2

### Synthesis of 1-Benzyl-4-benzyloxy-2-ethyl-6-hydroxymethyl-1H-indole (13)

To a solution of 1.03 g (2.59 mmol) of compound (12) in tetrahydrofuran (22 ml) was added at 0°C 492 mg (13.0 mmol) of lithium aluminum hydride under nitrogen atmosphere and the resulting mixture was stirred at the same temperature for 30 minutes. To the reaction solution were successively added gradually at 0°C methanol, water, and dilute hydrochloric acid. After extraction with ethyl acetate, the organic layer was washed successively with water twice and an aqueous saturated solution of sodium chloride, then dried over anhydrous sodium sulfate, and concentrated. The thus-obtained crude product was recrystallized from ethyl acetate - n-hexane to obtain 922 mg (96%) of the objective compound (13).
Melting point: 135-136 °C
IR ν max (Nujol) 3292, 1584, 1541 cm⁻¹
¹H NMR (CDCl₃) δ 1.31 (t, J = 7.5 Hz, 3H), 2.61-2.70 (m, 2H), 4.69 (s, 2H), 5.24 (s, 2H), 5.30 (s, 2H), 6.49 (s, 1H), 6.63 (s, 1H), 6.86 (s, 1H), 6.90-6.97 (m, 2H), 7.18-7.30 (m, 3H), 7.32-7.45 (m, 3H), 7.50-7.56 (m, 2H).

| Elemental analysis (%) for C₂₅H₂₅NO₂·0.2 H₂O: | | | |
|---|---|---|---|
| Calcd.: | C, 80.06; | H, 6.83; | N, 3.73 |
| Found: | C, 80.23; | H, 6.83; | N, 3.83 |

### Step 3

### Synthesis of 6-Acetoxymethyl-1-benzyl-4-benzyloxy-2-ethyl-1H-indole (14)

To a solution of 922 mg (2.48 mmol) of compound (13) in pyridine (2.5 ml) was added at 0°C 468 µl (4.96 mmol) of acetic anhydride under nitrogen atmosphere and the resulting mixture was stirred at room temperature for 4 hours. The reaction solution was partitioned in a mixture of ethyl acetate and 10% hydrochloric acid. The organic layer was washed successively with 10% hydrochloric acid, water twice, and an aqueous saturated solution of sodium chloride, then dried over anhydrous sodium sulfate, and concentrated. The thus-obtained crude product was recrystallized from ethyl acetate - n-hexane to obtain 984 mg (96%) of the objective compound (14).
Melting point: 115-116 °C
IR ν max (Nujol) 1725, 1586, 1540 cm⁻¹
¹H NMR (CDCl₃) δ 1.30 (t, J = 7.5 Hz, 3H), 2.05 (s, 3H), 2.65 (dq, J = 0.9, 7.5 Hz, 2H), 5.11 (s, 2H), 5.23 (s, 2H), 5.30 (s, 2H), 6.49 (d, J = 0.9 Hz, 1H), 6.61 (d, J = 0.9 Hz, 1H), 6.87 (s, 1H), 6.91-6.98 (m, 2H), 7.19-7.30 (m, 3H), 7.32-7.46 (m, 3H), 7.50-7.58 (m, 2H).

| Elemental analysis (%) for C₂₇H₂₇NO₃: | | | |
|---|---|---|---|
| Calcd.: | C, 78.42; | H, 6.58; | N, 3.39 |
| Found: | C, 78.25; | H, 6.65; | N, 3.47 |

### Step 4

### Synthesis of 6-Acetoxymethyl-1-benzyl-4-benzyloxy-2-ethyl-3-oxamoyl-1H-indole (15)

To a solution of 394 µl (4.52 mmol) of oxalyl chloride in tetrahydrofuran (3 ml) was gradually added dropwise a solution of 935 mg (2.26 mmol) of compound (14) in tetrahydrofuran (15 ml) under nitrogen atmosphere at 0°C. After stirring at 0°C for 1 hour and then at room temperature for 2 hours and 15 minutes, the resulting solution was gradually added dropwise into a mixture of 1.51 ml (22.6 mmol) of 28% by weight of aqueous ammonia and 3 ml of tetrahydrofuran at 0°C. After stirring at 0°C for 30 minutes, the reaction solution was partitioned in a mixture of ethyl acetate and water. The organic layer was washed successively with water 6 times and an aqueous saturated solution of sodium chloride, then dried over anhydrous sodium sulfate, and concentrated. The thus-obtained crude product was recrystallized from ethyl acetate - n-hexane to obtain 1.03 g (94%) of the objective compound (15).
Melting point: 176-178 °C
IR ν max (Nujol) 3401, 3210, 1736, 1645 cm⁻¹
¹H NMR (CDCl₃) δ 1.18 (t, J = 7.5 Hz, 3H), 2.03 (s, 3H), 2.89 (q, J = 7.5 Hz, 2H), 4.62 (brs, 1H), 5.08 (s, 2H), 5.15 (s, 2H), 5.35 (s, 2H), 6.01 (brs, 1H), 6.72 (s, 1H), 6.86 (s, 1H), 6.98-7.07 (m, 2H), 7.23-7.43 (m, 6H), 7.46-7.52 (m, 2H).

| Elemental analysis (%) for C₂₉H₂₈N₂O₅·0.2 H₂O: | | | |
|---|---|---|---|
| Calcd.: | C, 71.35; | H, 5.86; | N, 5.74 |
| Found: | C, 71.24; | H, 5.84; | N, 5.94 |

### Step 5

### Synthesis of 6-Acetoxymethyl-1-benzyl-2-ethyl-4-hydroxy-3-oxamoyl-1H-indole (16)

A mixture of 966 mg (1.99 mmol) of compound (15), 97 mg of 10% palladium on carbon, and 45 ml of ethyl acetate was subjected to catalytic reduction under hydrogen atmosphere. The reaction mixture was filtered and then concentrated. The thus-obtained crude product was subjected to silica gel chromatography (ethyl acetate - n-hexane = 1:4 to 1:2) followed by recrystallization from ethyl acetate - n-hexane to obtain 384 mg (49%) of the objective compound (16).
Melting point 161-162 °C
IR ν max (Nujol) 3403, 3158, 1737, 1689, 1585 cm⁻¹
¹H NMR (CDCl₃) δ 1.25 (t, J = 7.5 Hz, 3H), 2.05 (s, 3H), 3.02 (q, J = 7.5 Hz, 2H), 5.05 (s, 2H), 5.35 (s, 2H), 5.72 (brs, 1H), 6.40 (brs, 1H), 6.66 (s, 1H), 6.73 (s, 1H), 6.97-7.02 (m, 2H), 7.23-7.36 (m, 3H), 11.05 (s, 1H).

| Elemental analysis (%) for C₂₂H₂₂N₂O₅: | | | |
|---|---|---|---|
| Calcd.: | C, 66.99; | H, 5.62; | N, 7.10 |
| Found: | C, 67.02; | H, 5.63; | N, 7.05 |

### Step 6

### Synthesis of 6-Acetoxymethyl-1-benzyl-2-ethyl-4-methoxycarbonylmethyloxy-3-oxamoyl-1H-indole (17)

Compound (16) was reacted in the same manner as in step 6 in Example 1 to obtain the objective compound (17).
Melting point: 158-162 °C
IR ν max (Nujol) 3372, 3171, 1742, 1668, 1639 cm⁻¹
¹H NMR (CDCl₃) δ 1.19 (t, J = 7.5 Hz, 3H), 2.04 (s, 3H), 2.93 (q, J = 7.5 Hz, 2H), 3.80 (s, 3H), 4.75 (s, 2H), 5.07 (s, 2H), 5.36 (s, 2H), 5.41-5.57 (br, 1H), 6.50-6.66 (br, 1H), 6.56 (s, 1H), 6.90 (s, 1H), 6.98-7.07 (m, 2H), 7.22-7.34 (m, 3H).

| Elemental analysis (%) for C₂₅H₂₆N₂O₇·0.3 H₂O: | | | |
|---|---|---|---|
| Calcd.: | C, 63.63; | H, 5.68; | N, 5.94 |
| Found: | C, 63.70; | H, 5.59; | N, 5.84 |

### Step 7

### Synthesis of 1-Benzyl-4-carboxymethyloxy-2-ethyl-6-hydroxymethyl-3-oxamoyl-1H-indole (18)

Compound (17) was reacted using an aqueous solution of 3 molar equivalents of sodium hydroxide in the same manner as in step 8 in Example 1 to obtain the objective compound (18). (Yield: 83%).
Melting point (decomp.): 189 °C
IR ν max (Nujol) 3433, 3310, 3252, 1658, 1631 cm⁻¹
¹H NMR (d6-DMSO) δ 1.06 (t, J = 7.5 Hz, 3H), 2.88 (q, J = 7.5 Hz, 2H), 4.47 (s, 2H), 4.64 (s, 2H), 5.14 (brs, 1H), 5.50 (s, 2H), 6.53 (s, 1H), 6.97-7.06 (m, 3H), 7.21-7.38 (m, 3H), 7.40 (s, 1H), 7.75 (s, 1H), 12.50-13.10 (br, 1H).

| Elemental analysis (%) for C₂₂H₂₂N₂O₆·0.7 H₂O: | | | |
|---|---|---|---|
| Calcd.: | C, 62.46; | H, 5.58; | N, 6.62 |
| Found: | C, 62.47; | H, 5.46; | N, 6.83 |

### Experimental Example 1 Evaluation of Solubility in Water

A solution of 10.3 mg (24.3 µmol) of compound (11) in 4.85 ml (48.6 µmol) of a 0.01 M methanolic solution of sodium methylate was filtered through a Millipore filter and then concentrated. The resulting residue was dried under reduced pressure to obtain disodium salt (20). Dropwise addition of water by 10 µl to this disodium salt to dissolve resulted in the dissolution of the salt when 50 µl of water was added. The measurement under the same conditions was made on the monosodium salt of compound (10) as well as on the monosodium salt of compound (21), described in JP 7-285933A, for comparison, to determine the solubility in water (corrected for mg in 1 ml at 23°C). Compound (20) and compound (21) (reference compound) are illustrated below.

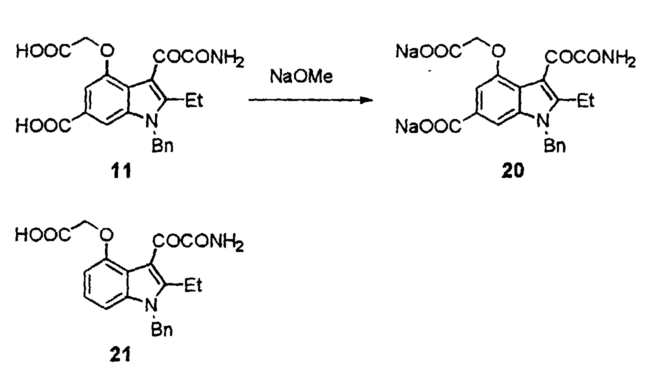

The results are shown in Table 1.

**Table 1**

| Compound No. | Solubility of Na salt in water (23°C) (mg/ml) |
|---|---|
| Compound of the present invention | |
| 10 | 68 |
| 11 | 228 |

| Reference compound | |
|---|---|
| 21 | 1.4 |

The above results indicate that the compounds of the present invention are more soluble in water as compared with the reference compound.

### Experimental Example 2 In vitro Test for sPLA₂-Inhibitory Action by PC/DOC Method

The inhibitory activity against sPLA₂ (secretory PLA₂) was determined according to the method of Schädlich, H. R., Büchler, M. et al. described in J. Clin. Chem, Chin. Biochem. 25: 505-609 (1987).

### Preparation of Aqueous Substrate Solution

A 50 mM solution of 1-palmitoyl-1-oleoyl-sn-glycero-3-phosphatidylcholine in chloroform (PC) was used as a substrate. A 150 mM solution of sodium deoxycholate in methanol (DOC) was used as a surface-active agent

For each of test compounds (compounds (10) and (11)) were used 2 µl each of the substrate and the surface-active agent. First, the substrate and the surface-active agent were transferred into an Eppendorf tube and the solvent was evaporated by applying a nitrogen stream. Then, the tube was placed in a desiccator and evacuated with a pump to complete dryness. Thereto was added water in an amount to be used for the assay and the solution was stirred for 1 minute. The final concentrations were adjusted so as to make PC/DOC = 1 mM/3 mM.

### Assay Method

### Preparation of Buffer Solution for Measurement

As the buffer solution for measurement was prepared a Tris buffer solution (50 mM, pH 8.0) containing 150 mM sodium chloride, calcium chloride (10 mM), and bovine serum albumin (1 mg/ml).

To a test compound solution were added the substrate solution and the buffer solution for measurement prepared as described above and the resulting mixture was stirred to mix. After a preincubation at 40°C for 10 minutes, human sPLA₂ was added and the enzymatic reaction was carried out at 40°C for 30 minutes. Then, 400 µl of the Dole's reagent (isopropanol:n-heptane: 2N sulfuric acid = 40:10:1) was added to stop the enzymatic reaction. After 12 µl of a margarine acid solution (C17.0, 0.5 nmol/µl) was added as an internal standard for HPLC analysis, distilled water (160 µl) and n-heptane (240 µl) were added. After stirring for about 10 seconds to extract the fatty acid, the reaction product 200 µl of the supernatant heptane layer was mixed with about 80 mg of silica gel to remove the contaminated, unchanged substrate by adsorption. Then, 150 µl of the heptane layer was separated by centrifugation and transferred into a separate Eppendorf tube which was placed in a desiccator for evaporation of the solvent. Thereto was added 100 µl of the ADAM reagent (9-anthryldiazomethane at the final concentration of 0.05%: prepared by dissolution of the reagent in 100 µl of ethyl acetate, followed by addition of 900 µl of methanol and stirring) and the mixture was kept for 15 minutes to complete the esterification. HPLC analysis was carried out under the following analysis conditions:
- Column:: Supersphere ^{R}60 RP-8 (Merck & Co.)
- Mobile:: Phase: Acetonitrile : Distilled water (93:7)
- Detection:: Absorbance at 254 nm

The inhibitory activity of the compound was calculated from the amount of oleic acid thus-measured. The sPLA₂-inhibitory activity for each of the compounds was determined by the above-described method. Also, the determination under the same conditions was carried out for compound (21) for comparison. Table 2 shows the results.

**Table 2**

| Compound No. | IC₅₀ (nM) |
|---|---|
| Compound of the present invention | |
| 10 | 1.7 |
| 11 | 43.8 |

| Reference compound | |
|---|---|
| 21 | 13.6 |

The above results indicate that the compounds of the present invention exhibit almost equivalent sPLA₂-inhibitory activities as compared with the reference compound.

The pharmaceutical formulations shown in the following are only illustrative and are not intended to restrict the scope of the present invention. The term "Active ingredient" means a compound represented by formula (I) or a pharmaceutically permissible salt or a hydrate thereof.

### Formulation 1

| Ingredient | Amount (mg/capsule) |
|---|---|
| Active ingredient | 250 |
| Starch (dried) | 200 |
| Magnesium stearate | 10 |
| Total | 460 mg |

### Formulation 2

| Ingredient | Amount (mg/tablet) |
|---|---|
| Active ingredient | 250 |
| Cellulose (microcrystalline) | 400 |
| Silicon dioxide (fumed) | 10 |
| Stearic acid | 5 |
| Total | 665 mg |

### Formulation 3

The formulation for intravenous injection is as follows:
- Active ingredient:: 100 mg
- Isotonic sodium chloride solution:: 1000 ml

The solution containing the above-described ingredients is intravenously administered to patients usually at a rate of 1 ml per minute.

### Industrial Applicability

As can be clearly seen from the above results, the compounds of the present invention possess potent sPLA₂-inhibitory activities and are highly water-soluble. Therefore, the compounds of the present invention are expected to be as highly useful pharmaceuticals for treatment of diseases which are considered to be caused by sPLA₂.

## Claims

1. A compound represented by the formula (I): 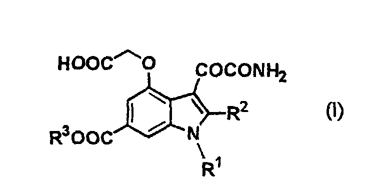 wherein R¹ is optionally substituted alkyl, optionally substituted cycloalkyl, optionally substituted aryl, optionally substituted aralkyl, optionally substituted heteroaryl, or optionally substituted heteroarylalkyl;
R² is hydrogen, optionally substituted alkyl, optionally substituted cycloalkyl, optionally substituted alkenyl, optionally substituted cycloalkenyl, -OR⁴, or - SR⁵ wherein R⁴ and R⁵ are alkyl or cycloalkyl; and
R³ is hydrogen, alkyl, or cycloalkyl, its ester, their pharmaceutically acceptable salts, or hydrate thereof.

2. The compound of claim 1, wherein R³ is hydrogen, methyl, ethyl, n-propyl, isopropyl, or cyclopropyl, its ester, their pharmaceutically acceptable salts, or hydrate thereof.

3. The compound of claim 2, wherein R¹ is aralkyl and R² is alkyl, its ester, their pharmaceutically acceptable salts, or hydrate thereof.

4. The compound of claim 3, wherein R¹ is benzyl and R² is ethyl, its ester, their pharmaceutically acceptable salts, or hydrate thereof.

5. A pharmaceutical composition which contains a compound, its ester, their pharmaceutically substituted acceptable salts, or hydrate thereof of any one of claims 1 to 4 as an active ingredient.

6. A composition for inhibiting sPLA₂ which contains a compound, its ester, their pharmaceutically substituted acceptable salts, or hydrate thereof of any one of claims 1 to 4 as an active ingredient.
